(19) Europäisches Patentamt
European Patent Office
Office européen des brevets

(11) Publication number: 0 059 606
A1

(12) **EUROPEAN PATENT APPLICATION**

(21) Application number: 82300966.7

(22) Date of filing: 25.02.82

(51) Int. Cl.³: C 07 D 495/04
A 61 K 31/41
//(C07D495/04, 249/00, 335/00)

(30) Priority: 03.03.81 GB 8106629
27.11.81 GB 8135950

(43) Date of publication of application:
08.09.82 Bulletin 82/36

(84) Designated Contracting States:
BE CH DE FR GB IT LI NL

(71) Applicant: BEECHAM GROUP PLC
Beecham House Great West Road
Brentford Middlesex(GB)

(72) Inventor: Buckle, Derek Richard
18 Hillfield Road
Redhill Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Benzothiopyrano(2,3-d)-v-triazole derivatives and pharmaceutical compositions containing them.**

(57) Compounds of formula (I):

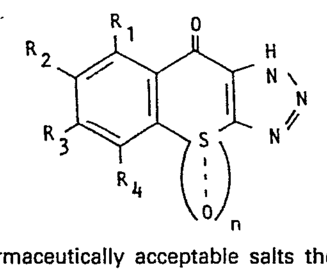

(1)

and pharmaceutically acceptable salts thereof, wherein each of $R_1$, $R_2$, $R_3$ and $R_4$, which may be the same or different, represents a hydrogen or halogen atom, a nitro, lower alkyl or lower alkoxy group, or any adjacent two of $R_1$ to $R_4$ taken together represent an alkylene group containing from 3 to 5 carbon atoms or a 1,4-buta-1,3-dienylene group, n is 0, 1 or 2, and the S---O bond represents a single dative bond when n is 1 and a double bond when n is 2; are useful in the prophylaxis and treatment of diseases whose symptoms are controlled by the mediators of the allergic response, for example, asthma, hay fever, rhinitis and allergic eczema.

EP 0 059 606 A1

0059606

TITLE MODIFIED
see front page

NOVEL COMPOUNDS

This invention relates to novel compounds having anti-allergy activity, to a process for their preparation, and to pharmaceutical compositions containing them.

It is generally recognised that certain cells, eg mast cells are activated by antibody-antigen combinations and release substances which mediate an allergic response. In European Patent Application No 79301318.6 (Publication No 0007727) is disclosed a class of 9-oxo-1H,9H-benzopyrano[2,3-d]-v-triazole derivatives which inhibit this type of antigen-induced response in mammals, and are therefore of value in the prophylaxis of diseases in which the symptoms are controlled by mediators of the allergic response. Examples of such diseases include bronchial asthma, rhinitis, hayfever and allergic eczema.

The compounds disclosed in the said European Patent Application have the formula (A):

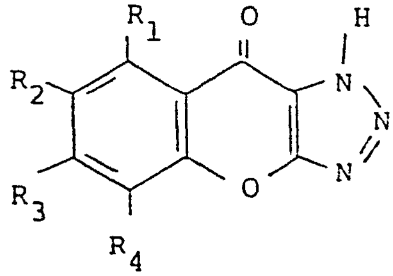

(A)

and pharmaceutically acceptable salts thereof, wherein $R_1$, $R_2$, $R_3$ and $R_4$, which may be the same or different, represent hydrogen, halogen, nitro, lower alkyl or lower alkoxy, or any adjacent two of $R_1$ to $R_4$ taken together represent an alkylene group containing from 3 to 5 carbon atoms or a 1,4-buta-1,3-dienylene group.

A novel class of compounds structurally distinct from the compounds of formula (A) has now been discovered. These new compounds have anti-allergy activity of the type described above for the compounds of the formula (A).

Accordingly the present invention provides a compound of the formula (I):

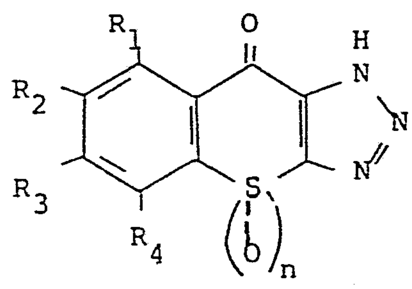

(I)

and pharmaceutically acceptable salts thereof, wherein $R_1$, $R_2$, $R_3$ and $R_4$ are as defined above in relation to a compound of formula (A), n is 0, 1 or 2, and the S---O bond represents a single dative bond when n is 1 and a double bond when n is 2.

By lower alkyl and lower alkoxy we mean such groups containing up to six carbon atoms.

Examples of suitable lower alkyl groups which $R_1$ to $R_4$ represent include methyl, ethyl and n-propyl, more suitably methyl.

Examples of suitable lower alkoxy groups which $R_1$ and $R_4$ represent include methoxy, ethoxy and n-propoxy.

Examples of suitable halogens which $R_1$ to $R_4$ represent include fluorine and chlorine.

Often $R_1$, $R_2$, $R_3$ and $R_4$ will be selected from hydrogen and lower alkyl.

Where compounds of formula (I) are highly substituted, it is appreciated that substituents $R_1$ to $R_4$ are selected for steric compatability.

The triazole moiety of the compounds of formula (I) has an acidic hydrogen, and accordingly may form salts. Examples of pharmaceutically acceptable salts falling within the scope of this invention include the aluminium, alkali metal and alkaline earth metal salts such as the sodium, potassium and magnesium salts; and salts with ammonia, organic bases and amino compounds.

Suitably in the compounds of the formula (I) at least one of $R_1$, $R_2$, $R_3$ and $R_4$ is hydrogen.

More suitably in the compounds of the formula (I) at least two of $R_1$, $R_2$, $R_3$ and $R_4$ are hydrogen.

Preferably in the compounds of the formula (I) $R_1$ and $R_4$ are hydrogen, and $R_2$ and $R_3$ are selected from methyl, ethyl and n- and iso-propyl.

In a further aspect of the invention, there is provided a compound of the formula (I) wherein the triazole moiety is protected with a protecting group. These compounds are not pharmaceutically active, but are useful as intermediates for the preparation of the compounds of formula (I). Suitable examples of protecting groups for the triazole moiety include labile benzyl groups such as $C_{1-6}$ alkoxy substituted benzyl groups, for example, p-methoxybenzyl groups.

The invention further provides a process for preparing a compound of formula (I) which process comprises the intramolecular cyclisation of a compound of formula (II):

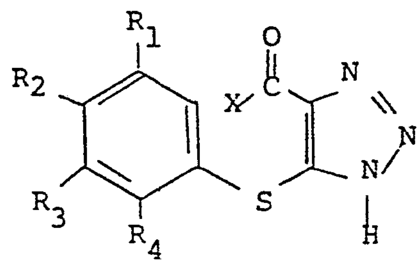

(II)

wherein $R_1$ to $R_4$ are as defined with reference to formula (I) above, the triazole moiety is optionally protected, and X is hydroxyl or an active substituent such that -COX is an acylating derivative, in the presence of a cyclising agent, and thereafter where desired or necessary de-protecting the triazole moiety, oxidising the resultant protected compound wherein n is 0 to the corresponding compounds wherein n is 1 or 2 and then deprotecting the triazole moiety, and/or salifying the product so obtained.

When X is hydroxyl, the cyclisation is suitably carried out in the presence of polyphosphoric acid or phosphorous pentoxide and methane sulphonic acid. In the case of polyphosphoric acid a diluent such as acetic acid may be used, and a temperature of 80-100°C is suitable.

Examples of active substituents include halides (ie X is halogen) particularly the chloride and bromide. Where X is a halogen the cyclising agent is suitably a Friedel Crafts catalyst, examples of which include aluminium chloride and stannic chloride, and the reaction is suitably carried out at around $0^{o}C$.

Where desired, the reaction may be carried out in the presence of a solvent or diluent which is inert to the reagents and products. Where the cyclisation is carried out using Friedel Crafts catalyst, suitable solvents or diluents include chlorinated alkanes such as methylene chloride, ethylene chloride and chloroform. When the cyclisation is carried out with polyphosphoric acid or phosphorous pentoxide and methane sulphonic acid, it is generally unnecessary to add a diluent.

As noted above, optionally the triazole moiety of the compound of formula (II) may be protected. We have found this particularly useful when a Friedel Crafts cyclisation reaction is used. Suitable examples of protecting groups for the triazole moiety are those mentioned above as suitable for protecting the compounds of formula (I) and include labile benzyl groups such as $C_{1-6}$ alkoxy substituted benzyl groups, for example p-methoxybenzyl groups. Such protecting groups may be removed after the cyclisation reaction in any convenient manner, for example by acid catalysis. It is preferable to use the p-methoxybenzyl protecting group which is readily removed after the cyclisation reaction using trifluoroacetic acid, the course of the cleavage being followed by hplc or NMR spectroscopy. Suitable temperatures of around 40-$70^{o}C$ can be used, with a suitable reaction time being around 2-8 hours. Other strong acids such as methane sulphonic acid behave similarly, but are less efficient.

On occasions, it will be found that the reaction conditions for the cyclisation reaction will be sufficient to effect the necessary deprotection after cyclisation without a further, separate, reaction step being required.

We would note that we have found it possible to use a benzyl protecting group, and to remove that group after the cyclisation reaction with sodium in liquid ammonia but we presently believe this route to be less preferred.

The compounds of formula (I) wherein n is 1 or 2 may be prepared from the corresponding N-protected compounds of the formula (IA):

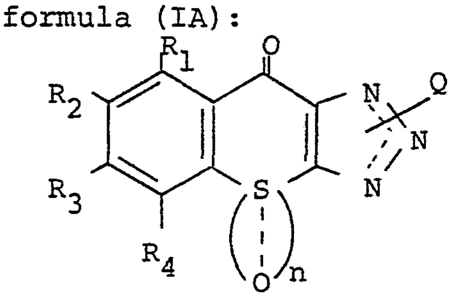

(IA)

wherein $R_1$ to $R_4$ are as defined in formula (I), n is 0 and Q is a protecting group,
by oxidation, followed by deprotection.

Preferably the protecting group is located at the N-3 atom on the triazole ring. The protection of the compound of formula (I) where n is 0 may be carried out by alkylating the said compound with a $C_{1-6}$ alkoxybenzyl halide, preferably p-methoxybenzyl chloride.

The oxidation conditions may be chosen as appropriate so as to give a mixture of compounds of formula (IA) wherein n is 1 or 2, or the compound wherein n is 2 alone. The latter may be achieved be oxidation with potassium permanganate. The mixture may be obtained by using milder oxidation conditions, for example, by oxidation with an organic peracid, suitably in an organic medium. The proportions of the two compounds in the mixture may be

adjusted by varying the concentrations of reactants and the reaction conditions. The mixture may be readily separated into its component compounds by conventional techniques such as liquid chromatography.

It will be appreciated from the above that the compounds of formula (IA) wherein n is 2 may be prepared from the corresponding compounds of formula (IA) wherein n is 1, by oxidation. After the oxidation the protecting group may be removed in the manner described above to give the compound of formula (I), preferably by using trifluoroacetic acid.

It will be appreciated that certain compounds of the formula (I) with non-symmetrical $R_1$ to $R_4$ substituents will give a mixture of isomers after the cyclisation step. Such isomers may, of course, be separated by conventional techniques, for example fractional recrystallisation and chromatography.

Salts of the compounds of formula (I) may be prepared in the usual manner, which is to react the free acid form of the compound of the formula (I) with the appropriate free base.

Compounds of formula (II) wherein -COX is an acylating derivative may be prepared from the corresponding compound of the formula (II) wherein X is OH in any of the usual ways of making a conversion of this kind. For example oxalyl chloride or thionyl chloride may be used to form an X is Cl compound.

Compounds of the formula (II) wherein X is OH may be prepared by the alkaline hydrolysis of a compound of formula (III), which like the compounds of formula (II) may optionally be protected:

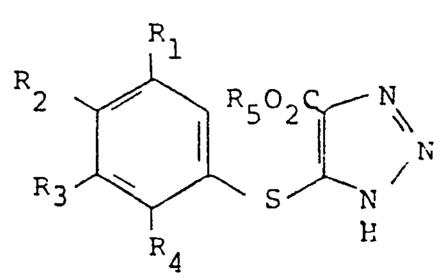

(III)

wherein $R_5$ is $C_{1-4}$ alkyl, such as ethyl.

Such hydrolysis may suitably be achieved with aqueous sodium hydroxide at a temperature of, for example, 40-100°C.

Compounds of the formula (III) may themselves be prepared by reacting a compound of formula (IV):

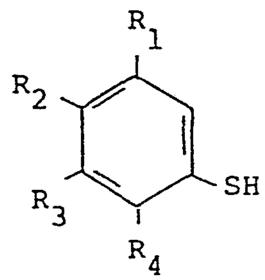

(IV)

with a compound of formula (V):

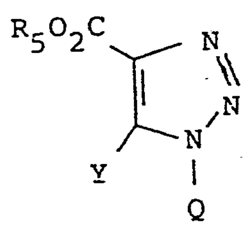

(V)

wherein Y is a leaving group such as a halo, for example chloro, and Q is a protecing group, such as those described previously; and thereafter if the desired compound of formula (III) is unprotected, removing the protecting group Q.

The reaction between compounds of formulae (IV) and (V) is suitably carried out by forming the anion of the compound of formula (IV) with a strong base such as sodium hydride, in N,N-dimethylformamide. Suitably this reaction is carried out at around 80°C for 6-24 hours.

The compounds of formulae (IV) and (V) are either known compounds, or may be prepared in analogous manner to known compounds.

The intermediates of formulae (II) and (III), and protected derivatives thereof, are believed to be novel, and thus as useful intermediates form an important aspect of this invention.

As noted hereinbefore, the compounds of the formula (I) and their salts may be used in the treatment of allergies, such as bronchial asthma, rhinitis, hayfever and allergic eczema.

In order to use the compounds of the invention as medicinal agents in this way, they will be formulated into pharmaceutical compositions in accordance with standard pharmaceutical procedure.

The invention also includes a pharmaceutical composition comprising a compound of formula (I) or a pharmaceutically acceptable salt thereof and a pharmaceutically acceptable carrier.

Compounds of formula (I) may be administered topically or systemically.

Topical formulations for administration to the skin include lotions and creams. Topical formulations for administration to the respiratory tract include solutions for application *via* a nebulizer or as an aerosol, or as a snuff or a microfine insufflatable powder. The active ingredient in an insufflatable powder has a small particle size i.e. less than 50 microns and preferably less than 10 microns. The active material is co-presented with a solid carrier such as lactose which has a particle size of less than 50 microns.

Systemic administration may be achieved by rectal, oral or parenteral administration. A typical suppository formulation comprises the active compound with a

binding agent and/or lubiricating agent such as gelatin or cocoa butter or other low melting vegetable waxes or fats. Typical parenteral compositions comprise a solution or suspension of the active material in a sterile aqueous carrier of parenterally acceptable oil.

Compounds of formula (I) which are active when given orally may be compounded in the form of syrups, tablets, capsules and lozenges. A syrup formulation will generally consist of a suspension or solution of the compound in a suitable liquid carrier such as ethyl alcohol, glycerine or water with a flavouring or colouring agent. Where the composition is in the form of a capsule, the solid in granular form optionally with a binding agent is encased in a gelatin shell. Where the composition is in the form of a tablet, any suitable pharmaceutical carrier routinely used for preparing solid formulations may be used. Examples of such carriers include magnesium stearate, starch, lactose, glucose, sucrose, rice flour and chalk. Preferably the composition is in unit dose form such as a pill, capsule or metered aerosol so that the patient may administer to himself a single dose.

Where appropriate, small amounts of anti-asthmatics and bronchodilators for example sympathomimetic amines such as isoprenaline, isoetharine, salbutamol, phenylephrine and ephedrine; xanthine derivatives such as theophylline and aminophylline; and corticosteroids such as prednisolone and adrenal stimulants such as ACTH may be included. As in common practice, the compositions will usually be accompanied by written or printed directions for use in the medical treatment concerned, in this case as an anti-allergic agent for treatment of, for example, asthma, hayfever or rhinitis.

In any of the foregoing formulations, a suitable dosage unit might contain from 1 to 500 mg of active ingredient. The effective dose of compound of formula (I) depends on the particular compound employed, the condition of the patient and on the frequency and route of administration, but in general is in the range from 0.01 mg/kg/day to 50 mg/kg/day of the patient's body weight.

The following Examples illustrate the preparation and properties of some compounds within the scope of this invention.

Example 1

a) Ethyl 5-hydroxy-1-(4-methoxybenzyl)-v-triazole-4-carboxylate

To a stirred solution of sodium (6.03 g; 0.26 mole) in ethanol (450 ml) was added diethyl malonate (41.9 g; 0.26 mole). After 30 minutes a solution of 4-methoxybenzyl azide (42.5 g; 0.26 mole) in ethanol (50 ml) was added dropwise to the stirred solution and the mixture gently refluxed for 18 hours. After cooling, the bulk of the ethanol was removed *in vacuo* and water added. Acidification gave a crystalline precipitate of the title compound which was filtered off, washed with water and dried. Recrystallisation from chloroform-petrol ether (40-60$^{o}$) gave 47.7 g (67%) of product of mp 117$^{o}$C.

(Found: C, 56.41; H, 5.74; N, 15.02; $C_{13}H_{15}N_3O_4$ requires C, 56.31; H, 5.45; N, 15.15%).

b) Ethyl 5-chloro-1-(4-methoxybenzyl)-v-triazole-4-carboxylate

Phosphorus pentachloride (30 g; 0.144 mole) was added to a stirred solution of ethyl 5-hydroxy-1-(4-methoxybenzyl)-v-triazole-4-carboxylate (37 g; 0.133 mole) in dry toluene (400 ml) and the mixture was warmed to $40^{o}$ (oil bath temperature) for 90 minutes. The solvent was evaporated *in vacuo* and the residue taken up in ether and washed well with saturated aqueous sodium bicarbonate solution and water. Evaporation of the dried organic phase gave an oil from which the chloro compound, 18 g (46%) of mp $74^{o}C$ was isolated by crystallisation from ether-petrol ($40-60^{o}$).

(Found: C, 52.59; H, 4.68; N, 13.90; Cl, 11.96; $C_{13}H_{14}ClN_3O_3$ requires C, 52.79; H, 4.77; N, 14.21; Cl, 11.99%).

c) Ethyl 1-(4-methoxybenzyl)-5-phenylthio-v-triazole-4-carboxylate

Finely powdered ethyl 5-chloro-1-(4-methoxybenzyl)-v-triazole-4-carboxylate (7.86 g, 0.0266 mole) was added to a solution of the sodium salt of thiophenol (from sodium hydride [1.28 g of a 50% dispersion in mineral oil] and thiophenol [2.92 g, 0.0266 mole])in dry N,N-dimethylformamide (70 ml) and the mixture stirred overnight at 50°C. The solvent was removed *in vacuo* and the residue partitioned between water and ethyl acetate. The organic phase was washed with dilute sodium hydroxide solution, water and brine and dried ($MgSO_4$). Evaporation gave an oil which afforded 7.01 g (71%) of material of mp (ether-light petroleum) 60-61°C after chromatography on silica and elution with chloroform. $\nu_{max}$ (mull) 1240, 1260, 1520, 1618, 1730 $cm^{-1}$; $\delta$ ($CDCl_3$): 1.29 (3H, t, J 7 Hz); 3.76 (3H, s); 4.32 (2H, q, J 7Hz); 5.50 (2H, s); 6.71 (2H, d, J 9Hz); 7.12 (7H, m); $M^+$ 369.1133 ($C_{19}H_{19}N_3SO_3$) (Found: C, 61.7; H, 4.85; N, 11.1; S, 8.45; $C_{19}H_{19}N_3SO_3$ requires: C, 61.75; H, 5.2; N, 11.35; S, 8.7%).

d) 1-(4-Methoxybenzyl)-5-phenylthio-v-triazole-4-carboxylic acid

Ethyl 1-(4-methoxybenzyl)-5-phenylthio-v-triazole-4-carboxylate (6.65 g, 0.018 mole) and 1.25M aqueous sodium hydroxide (32 ml) were stirred at 70°C for 3 hours and the resulting clear solution was cooled and acidified. The precipitated oil was extracted into ethyl acetate and the dried ($MgSO_4$) extracts evaporated to 6.13 g (100%) of crystalline solid. Recrystallisation from ethyl acetate gave mp 118-120°C (dec). $\nu_{max}$ (mull) 1260, 1518, 1535, 1590, 1618, 1705, 2600 (broad) $cm^{-1}$; δ (DMSO) 3.71 (3H, s); 5.58 (2H, s); 6.80 (2H, d, J 9Hz); 7.15 (7H, m); broad exchangeable signal at 11.5-14.5 δ; $M^+$ 341.0828 ($C_{17}H_{15}N_3O_2S$). (Found: C, 59.85; H, 4.1; N, 12.3; $C_{17}H_{15}N_3O_3S$ requires: C, 59.8; H, 4.45; N, 12.3%).

e) 3-(4-Methoxybenzyl)-9-oxo-9H-benzothiopyrano [2,3-d]-v-triazole

An excess of oxalyl chloride (11 g) was added to a solution of 1-(4-methoxybenzyl)-5-phenylthio-v-triazole-4-carboxylic acid (13.46 g, 0.039 mole) in dry dichloromethane (120 ml) followed by 2 drops of N,N-dimethylformamide. The mixture was stirred at ambient temperature for 1.5 hours and the solvent and excess oxalyl chloride removed in vacuo. The residual acid chloride ($\nu_{max}$ 1760 $cm^{-1}$) was dissolved in dry dichloromethane (150 ml), cooled to $0^{O}C$ and anhydrous aluminium chloride (20 g) added over 30 minutes at $0^{O}C$. The reaction mixture was stirred at this temperature for 3 hours and poured onto ice-hydrochloric acid. Extraction with chloroform gave, on evaporation of the dried extracts, a red oil which on chromatography* gave 5.14 g (40%) of product of mp (EtOH) $160^{O}C$ (dec). $\nu_{max}$ (mull) 1255, 1470, 1518, 1590, 1615, 1660 $cm^{-1}$; $\delta$ ($CDCl_3$) 3.78 (3H, s); 5.61 (2H, s); 7.11 (4H, AB quartet, J 8.5Hz; $\Delta\nu$ 25Hz); 7.54 (3H, broad); 8.68 (1H, broad); $M^+$ 323.0728 ($C_{17}H_{13}N_3O_2S$); $\lambda_{max}$ (EtOH) 237 (35,000); 242 (34,700); 332 (7,200) nm. (Found: C, 63.05; H, 3.6; N, 12.6; S, 9.9; $C_{17}H_{13}N_2SO_2$ requires: C, 63.15; H, 4.0; N, 13.0; S, 9.9%).

* on silica and elution with chloroform

f) 9-Oxo-1H,9H-benzothiopyrano[2,3-d]-v-triazole

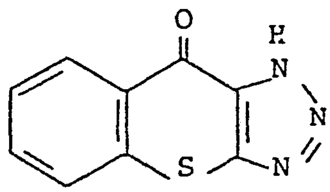

A solution of 3-(4-methoxybenzyl)-9-oxo-9H-benzothiopyrano[2,3-d]-v-triazole (0.96 g) in trifluoroacetic acid (50 ml) was stirred at 60-65°C for 5 hours when the deprotection was complete (by h.p.l.c.). The solvent was removed *in vacuo* and water added to the residue to give a yellow solid. Filtration and chromatography of this solid with chloroform+methanol on silica afforded 0.43 g (71%) of the title compound of mp (MeOH) 272-274°C (dec); $\nu_{max}$ (mull) 1485, 1595, 1620, 2600 (br); δ (DMSO) 7.80 (3H, complex m); 8.53 (1H, d.d); $M^+$ 203.0170 ($C_9H_5N_3SO$). (Found: C, 53.0; H, 2.45; N, 20.4; $C_9H_5N_3SO$ requires: C, 53.2; H, 2.5; N, 20.7%).

Example 2

a) 3-(4-Methoxybenzyl)-9-oxo-9H-benzothiopyrano [2,3-d]-v-triazole-4,4-dioxide

Potassium permanganate (5.6 g) was added in five portions over 2½ hours to a stirred solution of 3-(4-methoxybenzyl)-9-oxo-9H-benzothiopyrano[2,3-d]-v-triazole (2.2 g) in glacial acetic acid (150 ml) at 30-40$^{o}$C when reaction was complete. The solution was poured into water (500 ml) and decolourised by the dropwise addition of 30% hydrogen peroxide solution. The white solid which resulted was filtered off, washed well with water and dried to give 1.43 g (59%) of material of mp (EtOH) 154-155$^{o}$C. $\nu_{max}$ (mull) 1515, 1580, 1612, 1690 $cm^{-1}$; $\delta$ ($CDCl_3$) 3.79 (3H, s), 5.83 (2H, s); 7.21 (4H, AB quartet, J 9.7Hz; $\Delta\nu$ 57Hz); 7.95 (3H, m); 8.45 (1H, m); $M^+$ 355.0614 ($C_{17}H_{13}N_3O_4S$). (Found: C, 57.8; H, 3.85; N, 11.65; S, 9.35; $C_{17}H_{13}N_3O_4S$ requires: C, 57.45; H, 3.7; N, 11.85; S, 9.0%).

b) 9-Oxo-1H,9H-benzothiopyrano[2,3-d]-v-triazole-4,4-dioxide

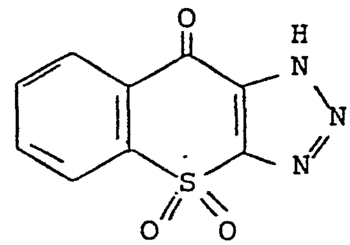

A solution of 3-(4-methoxybenzyl)-9-oxo-9H-benzothiopyrano[2,3-d]-v-triazole-4,4-dioxide (1.43 g) in trifluoroacetic acid (40 ml) was stirred for 3 hours at 65$^{o}$C and the solvent removed *in vacuo*. Addition of water to the residue gave a buff solid which after drying was chromatographed on silica eluting with chloroform to give 0.73 g(77%) of white solid of mp (EtOH-$H_2O$) 236-238$^{o}$C (dec); $\nu_{max}$ (mull) 1560, 1580, 1680, 2600 (br), 3280, 3520, 3570 $cm^{-1}$; $\delta$ (DMSO-$d_6$) 8.15 (4H, m); 9.10 (2H, broad exchangeable); $M^+$ 235.0060 ($C_9H_5N_3O_3S$); (Found: C, 44.1; H, 2.35; N, 17.05; $C_9H_5N_3SO_3.0.5H_2O$ requires: C, 44.25; H, 2.5; N, 17.2%).

Example 3

a) Ethyl 1-(4-Methoxybenzyl)-5-(4-methylphenylthio)-v-triazole-4-carboxylate

Ethyl 5-chloro-1-(4-methoxybenzyl)-v-triazole-4-carboxylate (20.6 g, 0.07 mole) was added to a stirred solution of the sodium salt of 4-toluenethiol (from 4-toluenethiol [8.7 g, 0.07 mole] and sodium hydride [3.36 g of a 50% dispersion of mineral oil]) in dry N,N-dimethylformamide (150 ml) and the mixture was stirred for 24 hours at $80^{o}C$. The solvent was removed in vacuo and the residue partitioned between ethyl acetate and water. Insoluble disulphide which separated at this stage was removed by filtration. The organic extracts were washed with dilute aqueous sodium hydroxide, water and brine and dried ($MgSO_4$). Evaporation afforded a crystalline solid which on recrystallisation from ethanol-petroleum [bp 60-$80^{o}C$] gave 18.33 g (68%) of product of mp 60-$61^{o}C$, $\nu_{max}$ 1518, 1530, 1612, 1730 $cm^{-1}$; $\delta$ ($CDCl_3$) 1.31 (3H, t, J 7Hz); 2.29 (3H, s); 3.78 (3H, s); 4.32 (2H, q, J7Hz); 5.51 (2H, s); 6.95 (4H, AB quartet, $\Delta\nu$ 34Hz, J 9Hz); 7.01 (4H, s); $M^+$ 383.1326 ($C_{20}H_{21}N_3O_3S$). (Found: C, 62.25; H, 5.75; N, 10.7; S, 8.3; $C_{20}H_{21}N_3O_3S$ requires: C, 62.65; H, 5.5; N, 10.95; S, 8.35%).

b) 1-(4-Methoxybenzyl)-5-(4-methylphenylthio)-v-triazole-4-carboxylic acid

A mixture of ethyl 1-(4-methoxybenzyl)-5-(4-methylphenylthio)-v-triazole-4-carboxylate (18.33 g, 0.048 mole), 1.25M aqueous sodium hydroxide solution (85 ml) and ethanol (50 ml) was stirred at 85°C for 3 hours and cooled. After breaking up the precipitated sodium salt the mixture was acidified with hydrochloric acid and the white product filtered off and dried to give 16.91 g (quantitative) of materia of mp (EtOAc) 154-155°C (dec), $\nu_{max}$ (mull) 1250, 1508, 1530, 1605, 1698, 2630 (br)$cm^{-1}$; δ (DMSO-$d_6$) 2.23 (3H, s); 3.70 (3H, s); 5.57 (2H, s); 6.93 (4H, AB quartet; J 8Hz, Δν 23Hz); 7.10 (4H, s); 1 low field broad exchangeable proton, $M^+$ 355.0968 ($C_{18}H_{17}N_3O_3S$). (Found: C, 60.8; H, 4.6; N, 11.65; S, 9.1; $C_{18}H_{17}N_3O_3S$ requires: C, 60.85; H, 4.8; N, 11.8; S, 9.0%).

c) 3-(4-Methoxybenzyl)-7-methyl-9-oxo-9H-benzothiopyrano[2,3-d]-v-triazole

To a solution of 1-(4-methoxybenzyl)-5-(4-methylphenylthio)-v-triazole-4-carboxylic acid (16.27 g, 0.045 mole) in dry dichloromethane (150 ml) was added an excess of oxalyl chloride (12.7 g) and 2 drops of N,N-dimethylformamide. The mixture was stirred at room temperature for 1.5 hours and then evaporated to dryness *in vacuo*. The crystalline acid chloride ($\nu_{max}$ 1610, 1765 $cm^{-1}$) was redissolved in dry dichloromethane (150 ml), cooled to $0^{o}C$ and anhydrous aluminium chloride (23.2 g) added over 30 minutes. After 3 hours stirring at $0^{o}C$ the product was poured onto ice-hydrochloric acid and then extracted with chloroform. Evaporation of the dried ($MgSO_4$) extracts afforded an oily solid which after chromatography on silica eluting with chloroform gave 8.93 g (59%) of yellow solid, mp (EtOH-$CHCl_3$) 193-194$^{o}C$, $\nu_{max}$ (mull) 1245, 1510, 1600, 1610, 1655 $cm^{-1}$; $\delta$ (DMSO-$d_6$) 2.48 (3H, s); 3.80 (3H, s); 5.80 (2H, s); 7.17 (4H, AB quartet, J 9Hz, $\Delta\nu$ 33Hz); 7.56 (1H, dd J 8Hz, 1.5Hz); 7.80 (1H, d, J 8Hz); 8.32 (1H, d, J 1.5Hz); $M^+$ 337.0875 ($C_{18}H_{15}N_3O_2S$). (Found: C, 63.7; H, 4.6; N, 12.1; N, 9.45; $C_{18}H_{15}N_3O_2S$ requires: C, 64.1; H, 4.5; N, 12.45; N, 9.5%).

d) 7-Methyl-9-oxo-1H,9H-benzothiopyrano[2,3-d]-v-triazole

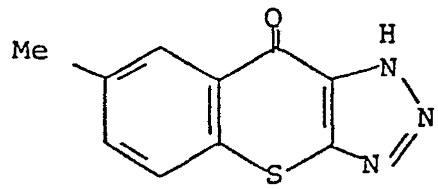

A solution of 3-(4-methoxybenzyl)-7-methyl-9-oxo-9H-benzothiopyrano[2,3-d]-v-triazole (1.5 g) in trifluoroacetic acid (50 ml) was stirred for 4 hours at 65$^{o}$C and the solvent removed *in vacuo*. Addition of water to the residue gave a yellow solid which was filtered off, dried and chromatographed with chloroform on silica to give 0.94 g (98%) of material of mp (EtOH) 270-272$^{o}$C (dec), $\nu_{max}$ (null) 1598, 1620, 2700 (br) $cm^{-1}$; δ (DMSO-$d_6$) 2.43 (3H, s); 7.54 (1H, d.d, J 8Hz, *ca* 2Hz); 7.80 (1H, d, J 8Hz); 8.27 (1H, diffuse d, J *ca* 2Hz); $M^+$ 217.0308 ($C_{10}H_7N_3OS$). (Found: C, 54.75; H, 3.4; N, 18.75; S, 14.3; $C_{10}H_7N_3OS.0.2H_2O$ requires: C, 54.4; H, 3.4; N, 19.05; S, 14.5%).

Example 4

a) 3-(4-Methoxybenzyl)-7-methyl-9-oxo-9H-benzothiopyrano[2,3-d]-v-triazole-4,4-dioxide

Potassium permanganate (5.6 g) was added in five portions over 1 hour to a warm (50 °C) solution of 7-methyl-3-(4-methoxybenzyl)-9-oxo-9H-benzothiopyrano [2,3-d]-v-triazole (2.4 g) in glacial acetic acid (150 ml). The product was then poured into water (500 ml) and decolourised by the addition of 30% hydrogen peroxide solution. The resulting white solid was filtered off, washed with water and dried to give 1.67 g of crude product. Chromatography on silica eluting with chloroform gave 1.57 g (60%) of material of mp (EtOH) 160-161°C; $\nu_{max}$ (mull) 1310, 1515, 1590, 1610, 1690 $cm^{-1}$; δ ($CDCl_3$) 2.56 (3H, s); 3.80 (3H, s); 5.82 (2H, s); 7.20 (4H, AB quartet, J 9Hz, Δν 52 Hz); 7.70 (1H, d.d. J 9Hz and ca 1-2Hz); 7.99 (1H, d, J 9Hz); 8.28 (1H, d, J ca 1-2Hz); $M^+$ 369.0789 ($C_{18}H_{15}N_3O_4S$). (Found: C, 58.45; H, 4.35; N, 11.35; S, 8.3; $C_{18}H_{15}N_3O_4S$ requires: C, 58.75; H, 4.1; N, 11.4; S, 8.7%).

b) 7-Methyl-9-oxo-1H, 9H-benzothiopyrano[2,3-d]-v-triazole-4,4-dioxide

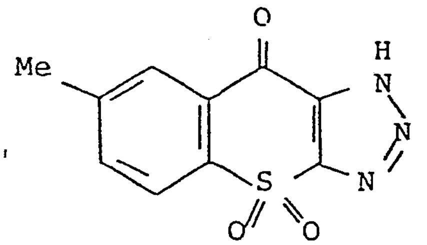

A solution of 3-(4-methoxybenzyl)-7-methyl-9-oxo-9H-benzothiopyrano[2,3-d]-v-triazole-4,4-dioxide (1.47 g) in trifluoroacetic acid (35 ml) was stirred at 60-65°C for 2.5 hours and the solvent evaporated *in vacuo*. Addition of water to the residue gave an off-white solid which was filtered off, washed with water and dried. Chromatography on silica eluting with chloroform gave 0.71 g (72%) of material of mp (EtOH) 254-255°C (dec); $\nu_{max}$ (mull) 1320, 1500, 1590, 1668, 3100 (br) $cm^{-1}$; δ (DMSO-$d_6$) 2.53 (3H, s); 7.88 (1H, d.d, J 8Hz, 1.5Hz); 8.12 (1H, d, J 1.5Hz); 8.13 (1H, d, J 8Hz); 10.36 (1H, broad exchangeable proton), $M^+$ 249.0209 ($C_{10}H_7N_3O_3S$). (Found: C, 47.9; H, 3.25; N, 16.6; S, 12.75; $C_{10}H_7N_3O_3S$ requires: C, 48.2; N, 2.85; N, 16.85; S, 12.85%).

Example 5

a) Ethyl 1-benzyl-5-phenylthio-v-triazole-4-carboxylate

Sodium hydride (3.24 g, 0.068M, of a 50% dispersion in mineral oil) was added to a stirred solution of thiophenol (7.44 g, 6.95 ml, 0.068 mole) in dry N,N-dimethylformamide (375 ml) and the mixture stirred for 1 hour. Ethyl 1-benzyl-5-chloro-v-triazole-4-carboxylate (18 g, 0.068 mole) was added and the reaction mixture was stirred at 90°C overnight. The solution after cooling was poured into water (1.5 l) and the product extracted into chloroform. The combined extracts were washed with brine, dried ($MgSO_4$) and evaporated to give a yellow oil. Trituration with petroleum ether (bp 40-60°C) gave solid material which recrystallised from petroleum ether (bp 60-80°C) to give 16 g (70%) of product of mp 45-47°C; $\nu_{max}$ (mull) 1720 $cm^{-1}$; δ 1.30 (3H, t, J 7.5 Hz); 4.35 (2H, q, J 7.5Hz); 5.58 (2H, s); 7.19 (10H, m). (Found: C, 63.35; H, 4.95; N, 12.35; S, 9.1; $C_{18}H_{17}N_3O_2S$ requires: C, 63.7; H, 5.05; N, 12.4; S, 9.45%)

b) 1-Benzyl-5-phenylthio-v-triazole-4-carboxylic acid

A mixture of ethyl 1-benzyl-5-phenylthio-v-triazole-4-carboxylate (16 g), 2.5M aqueous sodium hydroxide solution (60 ml) and water (130 ml) were stirred at 90-100$^{o}$C for 2 hours and the clear solution cooled to 0$^{o}$C. The sodium salt which separated was filtered off, re-suspended in water and acidified to give 12 g (82%) of the title acid of mp (EtOH) 78$^{o}$C; $\nu_{max}$ (mull) 1530, 1700, 2500, 3400, 3510 $cm^{-1}$; (Found: C, 58.6; H, 4.3; N, 12.85; S, 9.85; $C_{16}H_{13}N_3O_2S$.1$H_2O$ requires: C, 58.35; H, 4.6; N, 12.75; S, 9.7%).

c) 3-Benzyl-9-oxo-9H-benzothiopyrano[2,3-d]-v-triazole

A solution of 1-benzyl-5-phenylthio-v-triazole-4-carboxylic acid (12 g, 0.039 mole) in thionyl chloride (110 ml) was refluxed for 1.5 hours and the excess reagent removed in vacuo. The residual orange acid chloride was dissolved in dry dichloromethane (150 ml) and anhydrous aluminium chloride (16 g) added portionwise. The mixture was vigorously stirred at ambient temperature for 3 hours and then poured into water. Extraction with chloroform gave, after drying ($MgSO_4$), a yellow solid (9.0 g, 80%) which on recrystallisation from aqueous ethanol had mp 189-190°C; $\nu_{max}$ (mull) 1645 $cm^{-1}$; $\delta$($CDCl_3$) 5.77 (2H, s); 7.43 (5H, s); 7.70 (3H, m); 8.79 (1H, m); (Found: C, 65.6; H, 3.8; N, 14.25; S, 10.8; $C_{16}H_{11}N_3OS$ requires: C, 65.5; H, 3.8; N, 14.35; S, 10.9%).

d) 9-Oxo-1H,9H-benzothiopyrano[2,3-d]-v-triazole

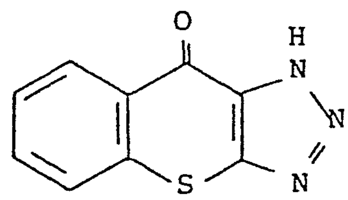

Sodium was added in small pieces to a solution of 1-benzyl-9-oxo-9H-benzothiopyrano[2,3-d]-v-triazole (1 g) in liquid ammonia (50 ml) until a permanent blue colour was obtained (time of addition=45 minutes) and ammonium chloride was then added. Evaporation of the ammonia gave a residue which was extracted with water and the filtered extracts acidified to give 0.33 g of impure product. Chromatography on silica afforded material identical with that in Example 1d).

Example 6

(a) Ethyl 5-(3,4-dimethylphenylthio)-1-(4-methoxybenzyl)-v-triazole-4-carboxylate

Powdered ethyl 5-chloro-1-(4-methoxybenzyl)-v-triazole-4-carboxylate (38.2 g, 0.129 mole) was added to a solution of the sodium salt of 3,4-dimethylthiophenol (from sodium hydride [6.20 g of a 50% dispersion in mineral oil] and the thiol [17.7 g, 0.129 mole]) in dry DMF (280 ml) and the mixture was stirred at 80$^{o}$C overnight. The solvent was evaporated *in vacuo* and the product partitioned between water and ethyl acetate. After filtration of a small amount of insoluble material the organic extracts were washed with water, brine and dried. Evaporation gave a solid which after recrystallization from ethanol gave 41.88 g (82%) of product of mp 98-100$^{o}$C, $\nu_{max}$ (mull) 1725, 1605 $cm^{-1}$, $\delta$ ($CDCl_3$) 1.30 (3H, t, J 7.5Hz), 2.10 (3H, s), 2.18 (3H, s), 3.77 (3H, s), 4.28 (2H, q, J 7.5Hz), 5.45 (2H, s), 6.88 (7H, m). Found: C, 63.18; H, 6.11; N, 10.62; S, 8.03; $C_{21}H_{23}N_3SO_3$ requires; C, 63.45; H, 5.83; N, 10.57; S, 8.07%).

(b) 5-(3,4-Dimethylphenylthio)-1-(4-methoxybenzyl)-v-triazole-4-carboxylic acid

Ethyl 5-(3,4-dimethylphenylthio)-1-(4-methoxybenzyl)-v-triazole-4-carboxylate (39.64 g, 0.1 mole), 1.25 M aqueous sodium hydroxide (170 mole) and ethanol (100 ml) were stirred at 85$^{o}$C for 2 hours and the clear solution cooled to 0$^{o}$C and acidified with hydrochloric acid. Extraction with ethyl acetate followed by drying and evaporation of the organic phase gave 36.9 g (100%) of title acid which recrystallized from ethyl acetate-petroleum ether [b.p. 40-60$^{o}$] to give material of mp 98-100$^{o}$C, $\nu_{max}$ (mull)

2600 (broad), 1698, 1615, 1525, 1510 $cm^{-1}$, $\delta$($DMSO-d_6$) 2.10 (3H,s), 2.17 (3H, s), 3.72 (3H, s), 5.60 (2H, s), 6.98 (7H, m), 13.4 (1H, broad exchangeable). Found: C, 61.53; H, 5.49; N, 11.42; S, 8.74; $C_{19}H_{19}N_3SO_3$ requires; C, 61.77; H, 5.18; N, 11.37; S, 8.68%).

(c) <u>6,7-Dimethyl-3-(4-methoxybenzyl)-9-oxo-9H-benzothiopyrano [2,3-d]-v-triazole and 7,8-dimethyl-3-(4-methoxybenzyl)-9-oxo-9H-benzothiopyrano[2,3-d]-v-triazole</u>

Excess oxalyl chloride (30 g) was added to a solution of 5-(3,4-dimethylphenylthio)-1-(4-methoxybenzyl)-v-triazole-4-carboxylic acid (35.56 g, 0.0955 mole) in dry dichloromethane (200 ml) and 2 drops of DMF were added. The mixture was stirred for 1½ hours at ambient temperature and the solution evaporated to a pale yellow solid ($\nu_{max}$ ( mull) 1760 $cm^{-1}$). After redissolution of this acid chloride in dry dichloromethane (300 ml) the solution was cooled to $0^oC$ and anhydrous aluminium chloride (48 g, 0.358 mole) was added portionwise with stirring over 45 minutes. The mixture was stirred for a further 3 hours at $0^oC$ and poured onto ice. Extraction with chloroform gave a dark solid which after filtration through 200 g of silica in chloroform gave 19.73 g (58%) of mixed isomers. Fractional recrystallization from ethanol-chloroform gave 5.76 g pure 6,7-dimethyl isomer of mp 220-222$^oC$, $\nu_{max}$ (mull) 1640, 1602, 1505 $cm^{-1}$, $\delta$($CDCl_3$) 2.32 (6H, s), 3.79 (3H, s), 5.60 (2H, s), 7.03 (4H, AB quartet, J 9Hz, $\Delta\nu$ 36Hz), 7.22 (1H, s), 8.39 (1H, s). Found; C, 65.01; H, 4.77; N, 11.53; S, 9.07; $C_{19}H_{17}N_3SO_2$ requires; C, 64.94; H, 4.88; N, 11.96; S, 9.12%).

Chromatographic separation of the enriched mother liquors on silica gel (Merck 7729) eluting with dichloromethane afforded the 7,8-dimethyl isomer as the faster running material. Recrystallisation from ethanol-chloroform gave pure product of mp 192-193$^{o}$C, $\nu_{max}$ (mull), 1655, 1615, 1520, 1260 cm$^{-1}$, δ ($CDCl_3$) 2.39 (3H, s), 2.88 (3H, s), 3.81 (3H, s), 5.61 (2H, s), 6.92 (2H, d, J 9Hz), 7.30 (4H, m).
Found: C, 64.60; H, 4.91; N, 11.65; S, 8.77; $C_{19}H_{17}N_3SO_2$ requires: C, 64.94; H, 4.88; N, 11.96; S, 9.12%.

(d) <u>6,7-Dimethyl-9-oxo-1H,9H-benzothiopyrano[2,3-d]-v-triazole</u>

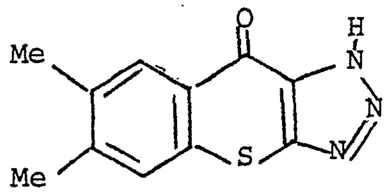

A solution of 6,7-dimethyl-3-(4-methoxybenzyl)-9-oxo-9H-benzothiopyrano[2,3-d]-v-triazole (1.5 g) in trifluoroacetic acid (40 ml) was stirred at 65-67°C for 3 hours and the solvent removed *in vacuo*. Water was added to the residue and the crystalline solid filtered off, washed and dried. Filtration through 15 g of silica, first with chloroform and then methanol-DMF gave 0.71 g (72%) of white solid of mp 274-276°C (dec.) after recrystallization from ethanol-DMF-water, $\nu_{max}$ (mull) 2900 (broad), 1615, 1590 $cm^{-1}$, $\delta$ (DMSO-$d_6$) 2.30 (6H,s), 7.59 (1H, s), 8.14 (1H, s). Found: C, 57.18; H, 3.62; N, 17.98; S, 13.81; $C_{11}H_9N_3SO$ requires; C, 57.13; H, 3.92; N, 18.17; 13.86%).

Example 7

(a) 6,7-Dimethyl-3-(4-methoxybenzyl)-9-oxo-9H-benzothiopyrano[2,3-d]-v-triazole-4,4-dioxide

To a solution of 6,7-dimethyl-3-(4-methoxybenzyl)-9-oxo-9H-benzothiopyrano[2,3-d]-v-triazole (2.46 g) in glacial acetic acid (200 ml) at 40-50°C was added a warm solution of potassium permanganate (1.12 g) in water (12 ml). Three further additions of the same quantity of oxidant were added over 1 hour, the course of the reaction being followed by hplc and tlc. After cooling, the mixture was poured into water (ca 650 ml) and decolourized with aqueous hydrogen peroxide. The white solid which separated was filtered off, washed well with water and dried to give 2.00 g (91%) of material of mp (ethanol-chloroform) 193-194°C, $\nu_{max}$ (mull) 1683, 1605, 1590, 1510 $cm^{-1}$, $\delta$(DMSO) 2.42 (3H, s), 2.47 (3H, s), 3.75 (3H,s), 5.88 (2H, s), 7.17 (4H, AB quartet, J 9Hz, $\Delta\nu$42Hz), 7.98 (1H, s), 8.07 (1H,s). Found: C, 59.26; H, 4.25; N, 10.84; S, 8.26; $C_{19}H_{17}N_3O_4S$ requires; C, 59.52; H, 4.47; N, 10.96; S, 8.36%.

(b) 6,7-Dimethyl-9-oxo-1H,9H-benzothiopyrano[2,3-d]-v-triazole-4,4-dioxide

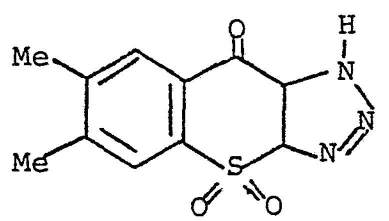

A solution of 6,7-dimethyl-3-(4-methoxybenzyl)-9-oxo-9H-benzothiopyrano[2,3-d]-v-triazole-4,4-dioxide (2.00 g) in trifluoroacetic acid (70 ml) was stirred at 65°C for 3.5 hours to effect deprotection. Evaporation of the cooled solution in vacuo gave a solid which was triturated

with water, filtered and dried and then chromotographed on silica eluting first with chloroform and then 5% methanol-chloroform to give 0.92 g (67%) of product of mp (ethanol) 262-263°C (dec), $\nu_{max}$ (mull), 3100, 1658, 1588, 1500 $cm^{-1}$, δ (DMSO) 2.41 (3H, s), 2.47 (3H, s), 7.98 (1H, s), 8.02 (1H, s), 13.1 (1H, broad exchangeable).

Found: C, 50.33; H, 3.77; N, 15.82; S, 11.90; $C_{11}H_9N_3O_3S$ requires; C, 50.18; H, 3.45; N, 15.96; S, 12.18%.

Example 8

(a) Ethyl 1-(4-methoxybenzyl)-5-(4-methoxyphenylthio)-v-triazole-4-carboxylate

Powdered ethyl 5-chloro-1-(4-methoxybenzyl)-v-triazole-4-carboxylate (20.6 g, 0.07 mole) was added to a solution of the sodium salt of 4-mercaptoanisole (from sodium hydride [3.36 g of a 50% dispersion in mineral oil] and thiol [9.8 g, 0.07 mole]) in dry DMF (150 ml) and the mixture was stirred at 80°C overnight. The DMF was evaporated in vacuo and the residue partitioned between water and ethyl acetate (insoluble material was filtered off). The organic phase was washed well with dilute aqueous sodium hydroxide, water and brine and dried ($MgSO_4$). Evaporation afforded a white crystalline solid which was recrystallized from ethanol-petroleum ether [b.p. 60-80°C] to give 20.75 g (74%) of material of mp 95-96°C, $\nu_{max}$ (mull) 1730, 1615, 1595, 1520, 1500 $cm^{-1}$, $\delta$ (DMSO) 1.34 (3H, t, J 7Hz), 3.78 (6H, s), 4.37 (2H, q, J 7Hz), 5.56 (2H, s), 6.72 (2H, d, J 9Hz), 6.80 (2H, d, J 9Hz), 7.10 (2H, d, J 9Hz), 7.13 (2H, d, J 9Hz).
(Found: C, 60.36; H, 5.57; N, 10.46; S, 8.07; $C_{20}H_{21}N_3SO_4$ requires; C, 60.13; H, 5.30; N, 10.52; S, 8.03%).

(b) Ethyl 5-(4-methoxyphenylthio)-1H-v-triazole-4-carboxylate

A solution of ethyl 1-(4-methoxybenzyl)-5-(4-methoxyphenylthio)-v-triazole-4-carboxylate (2.00 g) in trifluoroacetic acid (50 ml) was stirred at 65°C For 2.5 hours and the solvent evaporated in vacuo and water added. Extraction with chloroform and evaporation of the dried extracts afforded crude product as a red oil. Chromatography on

silica eluting with chloroform gave 1.39 g (99%) of material as a pale coloured gum, $\nu_{max}$ ($CHCl_3$) 3120 (broad), 1715, 1590 $cm^{-1}$, $\delta$($DCl_3$) 1.40 (3H, t, J 7.5Hz), 3.80 (3H, s), 4.42 (2H, q, J 7.5Hz), 7.27 (4H, AB quartet, J 9.5Hz, $\Delta\nu$54Hz), 1 broad low field exchangeable proton. $M^+$ 279.0681 ($C_{12}H_{13}N_3SO_3$).

(c) <u>5-(4-Methoxyphenylthio)-1H-v-triazole-4-carboxylic acid</u>

A mixture of ethyl 5-(4-methoxyphenylthio)-1H-v-triazole-4-carboxylate (11.15 g, 0.040 mole) and 1.25M aqueous sodium hydroxide (160 ml) was stirred for 3 hours at 80°C and the clear solution cooled to 0°C. Acidification afforded the title product as a white crystalline solid which was separated and dried to give 9.01 g (90%) of material of mp (ethyl acetate-petroleum ether [b.p. 40-60°] 165°C (dec), $\nu_{max}$(mull), 3100 (broad), 2650 (broad), 1680, 1590, 1565 $cm^{-1}$, $\delta$(DMSO-$d_6$) 3.90 (3H, s), 7.35 (4H, AB quartet, J 8.5Hz, $\Delta\nu$40Hz), 14.5 (2H, broad exchangeable). Found: C, 47.91; H, 3.69; N, 16.70; S, 12.77; $C_{10}H_9N_3O_3S$ requires; C, 47.80; H, 3.61; N, 16.72; S, 12.76%.

(d) 7-<u>Methoxy-9-oxo-1H,9H-benzothiopyrano[2,3-d]-v-triazole</u>

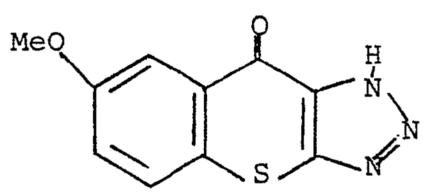

To a solution of phosphorus pentoxide (60 g) in 98% methanesulphonic acid (150 g) at 80°C was added powdered 5-(4-methoxyphenylthio)-1H-v-triazole-4-carboxylic acid (7.78 g, 0.o31 mole) and the solution was stirred at 103°C for 24 hours. The cooled mixture was diluted with 600 ml of ice-water, left for 1.5 hours and the solid filtered. Chromatography on silic gel eluting with chloroform gave

0.68 g (9.5%) of pale yellow solid. Recrystallization from ethanol-DMF gave a lemon yellow solid of mp 275-277$^{O}$C (dec), $\nu_{max}$ (mull) 2400 (broad), 1622, 1600, 1480, 1290 $cm^{-1}$, $\delta$ (DMSO-$d_6$) 3.89 (3H, s), 7.41 (1H, d.d, J 8.5Hz, 3Hz), 7.90 (1H, d, J 8.5Hz), 7.82 (1H, d, J 3Hz). $M^+$ 233.0255 ($C_{10}H_7N_3O_2S$).

Found; C, 50.47; H, 3.19; N, 17.65; S, 13.84; $C_{10}H_7N_3SO_2$ 0.25 $H_2O$ requires; C, 50.52; H, 3.18; N, 17.67; S, 13.49%.

Example 9

(a) 9-Oxo-3-(4-methoxybenzyl)-9H-benzothiopyrano[2,3-d]-v-triazole-4-oxide and 9-oxo-3-(4-methoxybenzyl)-9H-benzothiopyrano[2,3-d]-v-triazole-4,4-dioxide

To a solution of 9-oxo-3-(4-methoxybenzyl)-9H-benzothiopyrano[2,3-d]-v-triazole (1.615 g, 5 mmole) in chloroform (125 ml) at $0^{o}C$ was added a solution of m-chlorperbenzoic acid (1.015 g, 5 mmole of 85% purity) in chloroform (20 ml) and the mixture was stirred at room temperature. After 3 days a further 0.25 g of peracid was added and the reaction left for a further 3 days after which time the solvent was removed in vacuo and the residue chromatographed on silica eluting with dichloromethane. The sulphone, 0.60 g (34%) of mp 157-158$^{o}C$ (from ethanol chloroform) eluted first followed by 1.04 g (61%) of isomeric sulphoxides of mp 158-159$^{o}C$, $\nu_{max}$ (mull) 1693, 1610, 1590, 1515, 1258 $cm^{-1}$, $\delta$ ($CDCl_3$) 3.80 (3H, s), 5.71 (1H, d, J 15Hz), 6.04 (1H, d, J 15Hz), 7.18 (4H, AB quartet, J 9Hz, $\Delta\nu$ 51Hz), 7.89 (3H, complex m), 8.50 (1H, complex m).

Recrystallization from ethanol-chloroform afforded material of mp 177-178$^{o}C$, thought to be a single isomer. Found: C, 60.27; H, 3.06; N, 12.22; S, 9.24; $C_{17}H_{11}N_3SO_3$ requires: C, 60.52; H, 3.29; N, 12.46; S, 9.50%.

(b) 9-Oxo-1H,9H-benzothiopyrano[2,3-d]-v-triazole-4-oxide

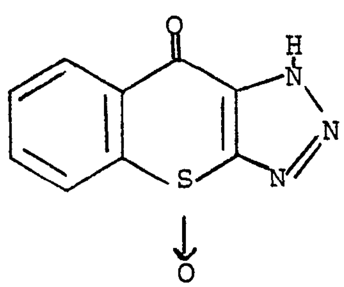

A solution of 9-oxo-3-(4-methoxybenzyl)-9H-benzothiopyrano[2,3-d]-v-triazole-4-oxide (1.00 g) in trifluoroacetic acid (30 ml) was stirred at 65°C for 6 hours when hplc suggested the absence of starting material. After cooling, the solvent was removed *in vacuo* and water was added. The solid was filtered off, washed with water, dried and chromatographed on silica eluting first with chloroform and then 10% methanol in chloroform to give 0.23 g (36%) of title compound of mp 174°C (dec) from ethanol, δ($DMSO-d_6$) 6.3 (2H, broad, exchangeable) 8.1 (complex m). $M^+$ found 219.0104, calculated 219.0102.

Found: C, 48.37, H, 2.43; N, 18.35; $C_9H_5N_3SO_2$. 0.25 $H_2O$ requires; C, 48.32; H, 2.48 ; N, 18.78%

Example 10

7,8-Dimethyl-9-oxo-1H,9H-benzothiopyrano[2,3-d]-v-triazole

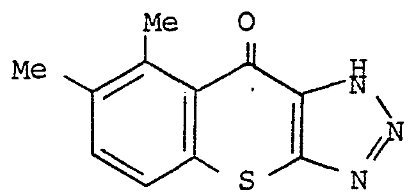

A solution of 7,8-dimethyl-3-(4-methoxybenzyl)-9-oxo-9H-benzothiopyrano[2,3-d]-v-triazole (0.57 g, from example 6c) in trifluoroacetic acid (15 ml) was stirred at 65°C for 4.5 hours and the solvent evaporated in vacuo. Water was added to the residue and the yellow solid was filtered off and washed well with water and dried. Chromatography on silica, eluting first with chloroform and then with 10% methanol in chloroform gave 0.38 g (100%) of title compound as a yellow solid. Recrystallisation from ethanol-chloroform (charcoal) gave off-white material of mp 283-285°C (dec), $\nu_{max}$ (mull) 2700 (broad), 1615, 1588, 1490, 1250 $cm^{-1}$; δ (DMSO-$d_6$), 2.43 (3H, s), 2.73 (3H, s), 6.62 (2H, AB quartet), 16.8 (1H, broad exchangeable). Found: C, 56.82; H, 4.03; N, 17.76; S, 13.67; $C_{11}H_9N_3SO$ requires: C, 57.13; H, 3.92; N, 18.17; S, 13.86%.

Example 11

a) 7,8-Dimethyl-3-(4-methoxybenzyl)-9-oxo-9H-benzothiopyrano[2,3-d]-v-triazole-4,4-dioxide

To a solution of 7,8-dimethyl-3-(4-methoxybenzyl)-9-oxo-9H-benzothiopyrano[2,3-d]-v-triazole (0.64 g, 1.8 mmole) in glacial acetic acid (50 ml) at 40-50°C was added a solution of potassium permanganate (292 mg) in water (3 ml). Three similar additions were made over ca 1 hour until no further starting sulphide remained by tlc. The product was poured into water (100 ml) and decolourised with 30% hydrogen peroxide. The white solid which remained was filtered off, washed with water and dried to give 0.46 (67%) of tlc pure material. Recrystallisation from ethanol gave product of mp 152-153°C, $\nu_{max}$(mull) 1688, 1620, 1525, 1325, 1265 $cm^{-1}$; δ ($CDCl_3$) 2.48 (3H, s), 2.78 (3H, s), 3.80 (3H, s), 5.82 (2H, s), 7.20 (4H, AB quartet, J 8Hz, Δν 54Hz), 7.65 (1H, d, J 7.5Hz), 7.91 (1H, d, J 7.5Hz).
Found: C, 59.54; H, 4.52; N, 10.73; S, 8.20; $C_{19}H_{17}N_3O_4S$.
requires: C, 59.52; H, 4.47; N, 10.96; S, 8.36%.

b) 7,8-Dimethyl-9-oxo-1H,9H-benzothiopyrano[2,3-d]-v-triazole-4,4-dioxide

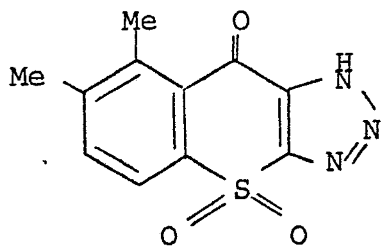

A solution of 7,8-dimethyl-3-(4-methoxybenzyl)-9-oxo-9H-benzothiopyrano[2,3-d]-v-triazole-4,4-dioxide (0.358 g) in trifluoroacetic acid (15 ml) was stirred at 65°C for 6 hours when deprotection was complete (by hplc). The trifluoroacetic acid was removed in vacuo and water was added. The white solid was filtered off, washed with water and dried. Chromatography on silica eluting with chloroform gave 0.245 g (100%) of material which on recrystallisation from aqueous ethanol had mp 271-272°C (dec),

$\nu_{max}$ (mull) 3200, 1682, 1565, 1290 $cm^{-1}$; δ (DMSO-$d_6$), 2.45 (3H, s), 2.69 (3H, s), 7.92 (2H, AB quartet, J 6.4Hz, Δν 14.4Hz), 11.15 (2H, broad exchangeable).

Found: C, 50.35; H, 3.66; N, 15.76; $C_{11}H_9N_3O_3S$ requires C, 50.18; H, 3.45; N, 15.96.

Passive Cutaneous Anaphylaxis (PCA)

Serum containing heat labile homocytotropic antibody was raised in rats to crystallised ovalbumin (Grade 3 Sigma) by the Method of I. Mota (Immunology 7, 681 (1964)) using Bordetella pertussis vaccine (4 x $10^{-10}$ organisms/ml Burroughs Welcome) as adjuvant.

Passive cutaneous anaphylaxis (PCA) was carried out by a method based on that of Ovary and Bier, (Proc. Soc. Exp. Bio. Med., 81, 584 (1952)) as modified by Goose and Blair (Immunology, 16, 749 (1969)) Male Sprague Dawley rats 230 - 340 gm were given 0.1 ml of each of six two fold serial dilutions of pooled antiserum in 0.9% saline (Polyfusor Boots) injected intradermally into separate sites on their shaved backs. 72 Hours later the rats were challenged by intravenous injection of 0.3 ml of a 1% solution of ovalbumin in isotonic saline buffered with 0.05M, pH 7.2 Sorenson Buffer (Bactohaemagglutination buffer Difco Laboratories), mixed with 0.2 ml of a 5% solution of Pontamine Sky Blue (6 BX C.1. 24410 Raymond A Lamb) in isotonic saline. The rats were killed after 20 minutes and the diameter of the blue wheals at the antibody injection sites was measured on the outer surface of the skin. The starting dilution of the serum was adjusted so that there was no response, after challenge, at the injection site of the highest dilution and a maximum response at the lowest dilutions. Typically, six twofold serial dilutions of the serum from 1/4 to 1/128 were used.

Compounds were tested for their ability to reduce the diameter of the wheals at those intradermal sites which in control animals gave less than maximum response. Each dose of the compounds was administered to

six rats at a measured time prior to intravenous challenge with ovalbumin. Control groups of six rats were given the same volume (0.2 ml: $100g^{-1}$) of carrier fluid at the same time prior to the challenge.

The results were calculated as follows: % inhibition of PCA = 100 (1 - a/b) where a = the sum of the diameters of the wheals produced in the test animal at the sites of antibody dilutions as used in control groups and b = the mean sum of the diameters of the wheals produced in the control group of animals at those antibody sites where at least five out of six of the animals gave less than maximum response. A typical variation in the control group of animals was SEM$\pm$ 6%.

| Compound of | Route | Carrier Fluid | Time* (mins) | Dose mg/kg | % Inhibition of rat PCA |
|---|---|---|---|---|---|
| Example 1 f | i.v. | PBS with $NaHCO_3$ | 0<br>0 | 1<br>10 | 35<br>90 |
| Example 2b | i.v. | PBS with $NaHCO_3$ | 0 | 10 | 68 |
| Example 3d | i.v. | PBS with $NaHCO_3$ | 0 | 10 | 42 |
| Example 4b | i.v. | PBS with $NaHCO_3$ | 0 | 10 | 65 |
| Example 6d | i.v. | PBS with $NaHCO_3$ | 0<br>0<br>0 | 0.5<br>1.0<br>2.0 | 65<br>86<br>90 |
| Example 7b | i.v. | PBS with $NaHCO_3$ | 0<br>0<br>0 | 0.5<br>1.0<br>2.0 | 56<br>59<br>74 |
| Example 8d | i.v. | PBS with $NaHCO_3$ | 0<br>0 | 0.5<br>2.0 | 34<br>58 |
| Example 9b | i.v. | PBS with $NaHCO_3$ | 0<br>0 | 0.5<br>2.0 | 30<br>61 |

* Time between administration of compound and antigen challenge

<u>Toxicity</u>

No toxic effects were observed in these tests.

CLAIMS

1. A compound of the formula (I):

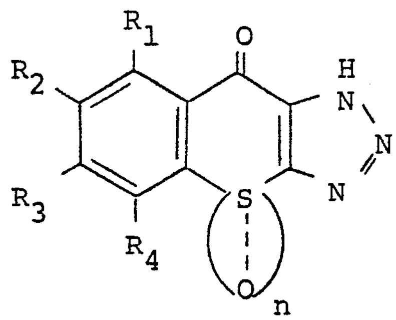

(I)

and pharmaceutically acceptable salts thereof, wherein each of $R_1$, $R_2$, $R_3$ and $R_4$, which may be the same or different, represents a hydrogen or halogen atom, a nitro, lower alkyl or lower alkoxy group, or any adjacent two of $R_1$ to $R_4$ taken together represent an alkylene group containing from 3 or 5 carbon atoms or a 1,4-buta-1,3-dienylene group, n is 0, 1 or 2, and the S---O bond represents a single dative bond when n is 1 and a double bond when n is 2.

2. A compound according to claim 1, in which each of $R_1$, $R_2$, $R_3$ and $R_4$ is a hydrogen atom or a lower alkyl group.

3. A compound according to claim 1 or claim 2, in which at least or two or $R_1$, $R_2$, $R_3$ and $R_4$ are hydrogen atoms.

4. A compound according to claim 3, in which $R_1$ and $R_4$ are both hydrogen atoms, and $R_2$ and $R_3$ are both lower alkyl groups.

5. A compound according to claim 1, selected from 9-oxo-1H,9H-benzothiopyrano[2,3-d]-v-triazole, 9-oxo-1H,9H-benzothiopyrano[2,3-d]-v-triazole-4,4-dioxide, 7-methyl-9-oxo-1H,9H-benzothiopyrano[2,3-d]-v-triazole, 7-methyl-9-oxo-1H,9H-benzothiopyrano[2,3-d]-v-triazole-4,4-dioxide, 6,7-dimethyl-9-oxo-1H,9H-benzothiopyrano[2,3-d]-v-triazole, 6,7-dimethyl-3-(4-methoxybenzyl)-9-oxo-9H-benzothiopyrano[2,3-d]-v-triazole-4,4-dioxide, 7-methoxy-9-oxo-1H,9H-benzothiopyrano[2,3-d]-v-triazole, 9-oxo-1H,9H-benzothiopyrano[2,3-d]-v-triazole-4-oxide, 7,8-dimethyl-9-oxo-1H,9H-benzothiopyrano[2,3-d]-v-triazole, and 7,8-dimethyl-9-oxo-1H,9H-benzothiopyrano[2,3-d]-v-triazole-4,4-dioxide.

6. A process for preparing a compound of formula (I), which comprises the intramolecular cyclisation of a compound of formula (II):

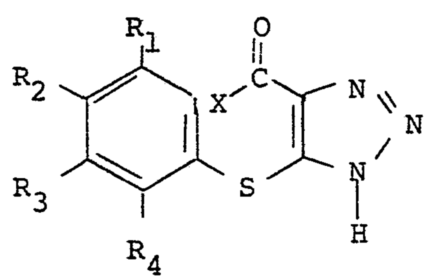

(II)

wherein $R_1$ to $R_4$ are as defined with reference to formula (I), the triazole moiety is optionally protected, and X is hydroxyl or an active substituent such that -COX is an acylating derivative, in the presence of a cyclising agent, and thereafter where desired or necessary de-protecting the triazole moiety, oxidising the resultant protected compound wherein n is 0 to the corresponding compounds

wherein n is 1 or 2, and then deprotecting the triazole moiety, and/or salifying the product so obtained.

7. A pharmaceutically composition comprising a compound of formula (I), or a pharmaceutically acceptable salt thereof, in combination with a pharmaceutically acceptable carrier.

8. A compound according to any one of claims 1 to 5, or a composition according to claim 7, for use in the treatment of allergies.

9. A compound according to any one of claims 1 to 5, in which the triazole moiety is protected with a protecting group.

European Patent Office

**0059606**
Application number
EP 82300966.7

# EUROPEAN SEARCH REPORT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 93, no. 19, November 10, 1980, Columbus, Ohio, USA<br>V.L. SAVAL'EV, O.S. ARTAMONOVA, T.G. AFANAS'EVA, V.A. ZAGOREVSKII "Synthesis of some benzopyrano- and thiopyranoazoles"<br>page 655, column 1, abstract no. 186 256m<br>& Khim. Geterotsikl. Soedin. 1980, (6), 853<br>* Formula II *<br>-- | 1 | C 07 D 495/04<br>A 61 K 31/41//<br>(C 07 D 495/04<br>C 07 D 249/00<br>C 07 D 335/00) |
| | | | **TECHNICAL FIELDS SEARCHED (Int.Cl. 3)** |
| A | GB - A - 1 410 311 (BAYER)<br>* Formula I *<br>---- | 1 | C 07 D 495/00<br>C 07 D 491/00 |

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| VIENNA | 03-05-1982 | HAMMER |

EPO Form 1503.1 06.78